# EUROPEAN PATENT SPECIFICATION

(11) **EP 2 611 355 B1**
(45) Date of publication and mention of the grant of the patent: **24.12.2014**
(21) Application number: 11769930.6
(22) Date of filing: 22.08.2011
(51) Int. Cl.: A61B 1/267

(54) **A LARYNGOSCOPE**
LARYNGOSKOP
LARYNGOSCOPE

(30) Priority: 02.09.2010 TR 201007321
(43) Date of publication of application: 10.07.2013
(73) Proprietor: Yilmaz, Taner, 06800 Ankara (TR)
(72) Inventor: Yilmaz, Taner, 06800 Ankara (TR)
(74) Representative: Dericioglu, E. Korhan
(86) International application number: PCT/IB2011/053670
(87) International publication number: WO 2012/028991

(56) References cited:
- WO-A1-2004/008951
- DE-A1- 3 934 804
- GB-A- 2 296 436
- US-A- 4 306 547
- US-B1- 6 471 643

## Description

### Technical Area

This invention is related to a laryngoscope used in endoscopic surgery of the larynx.

### Previous Technique

In the known technique of endoscopic surgery of the larynx, a laryngoscope is used to observe the lumen of the larynx. an intubation tube is placed inside the larynx to enable the patient's respiration during surgical intervention. This intubation tube remains outside and below the laryngoscope. In case of surgical interventions to the posterior half of the larynx, however, the intubation tube must be elevated anteriorly with the laryngoscope. During surgical intervention the intubation tube must stay above the laryngoscope. Because both intubation tube and laryngoscope are cylindirical in shape and because there is no structure to fix the intubation tube on the laryngoscope, the intubation tube slides from the top of laryngoscope and may fall to the posterior part of the larynx. In such a case, the operation must be paused and the intubation tube must be replaced again above the laryngoscope. Aforementioned procedures prolong the operation and sometimes even prevent the accomplishment of the operation.

In the known technique, United States patent document numbered US5665052 mentions about a laryngoscope and a multifunctional intubation stilet used during endoscopic procedure.

In another application of the known technique, international patent document numbered WO2006118984 mentions about a laryngoscope carrying intubation tube and a flexible imaging equipment. Because the intubation tube is connected to the laryngoscope it both gives a chance to the surgeon to inspect the larynx of the patient, and facilitates the surgeon's job in patients whose endoscopic procedures will be difficult to perform.

DE 39 34 804 A1 and WO 2004/008951 A1 discloses prior art laryngoscopes.

### Short description of the invention

The aim of this invention is to produce a laryngoscope which prevents the slipping of the intubation tube from above the laryngoscope during surgical procedures within the larynx.

Another aim of this invention is to produce a laryngoscope which enables the accomplishment of a surgical procedure to the posterior part of the larynx in a shorter time.

### Detailed description of the invention

A laryngoscope that is produced to accomplish the goal of this invention is shown in the attached figures. From these figures
Figure-1 is the side view of the laryngoscope which is the subject of this invention.
Figure-2 is the sectional view of the "A" detail of the laryngoscope which is the subject of this invention.

The parts in the figures were numbered one by one, and their equivalents were given below.
1. Laryngoscope
2. Body
3. Canal
4. Ridge
5. Burrow
6. Handle
7. Sleeve

The laryngoscope (1) which is the subject of this invention includes;
- a body (2) which is cylinder in shape and empty inside with one end placed inside the larynx.
- at least one handle (6) fixed with one tip to the other end of the body (2) and
- at least one sleeve (7) which is fixed to the other end of the handle (6) unconnected to the body (2) and facilitates the motion of the body (2).

The body (2) includes; at least a canal (3) which runs along the upper and outer wall of the body (2) starting from its tip placed within the larynx towards its end having the handle (6) and upon which the intubation tube is placed; at least one ridge (4) running along the outer side of the canal (3) and preventing the slippage of the intubation tube within the larynx; and at least two burrows (5) located on its end attached to the handle (6).

The canal (3) is as large as a portion of the surface of the intubation tube can lie upon, and can be located on the left, right or midline of the lateral wall of the body (2) depending on usage. In the preferred usage of the invention the canal (3) is semicircle in shape.

The ridge (4) runs along the canal (3) and is as long as the canal (3). The most superior end of the ridge (4) is on a level with the most superior end of the body (2) placed within the larynx. The ridge (4) fixes the laryngeal part of the intubation tube placed within the canal (3) and prevents the slippage of the intubation tube from over the body (2).

For the user to accomplish the surgical procedure comfortably, a light source is placed within one of the burrows (5) and aspirator cannula which functions to create vacuum is placed within the other burrow (5).

The handle (6) is used by the user to insert the laryngoscope (1) easily into the place where he would perform the surgical procedure easily, and to be able to change the direction of laryngoscope (1) in its place where the surgical procedure is carried out. performed. The sleeve (7), being structured for the user to introduce his finger, enables the user to move the laryngoscope (1) in vertical plane.

By introducing his fingers through the sleeve (7), the user grasps the handle (6) and inserts the body (2) inside the larynx. The user accomplishes the procedure easily because he can comfortably see inside the larynx with the help of light attached to the burrow (5) on the body (2). While the user is performing the aforesaid surgical operation, the intubation tube lies fixed on top of the body (2) and the problems such as slipping of the intubation tube are abolished. Thereby, the surgical procedure inside the larynx is accomplished easily.

The development of a wide variety of applications of the laryngoscope (1) which is the subject of this invention are possible. The invention cannot be restricted to the examples that are explained here. Basically it is as specified in the claims.

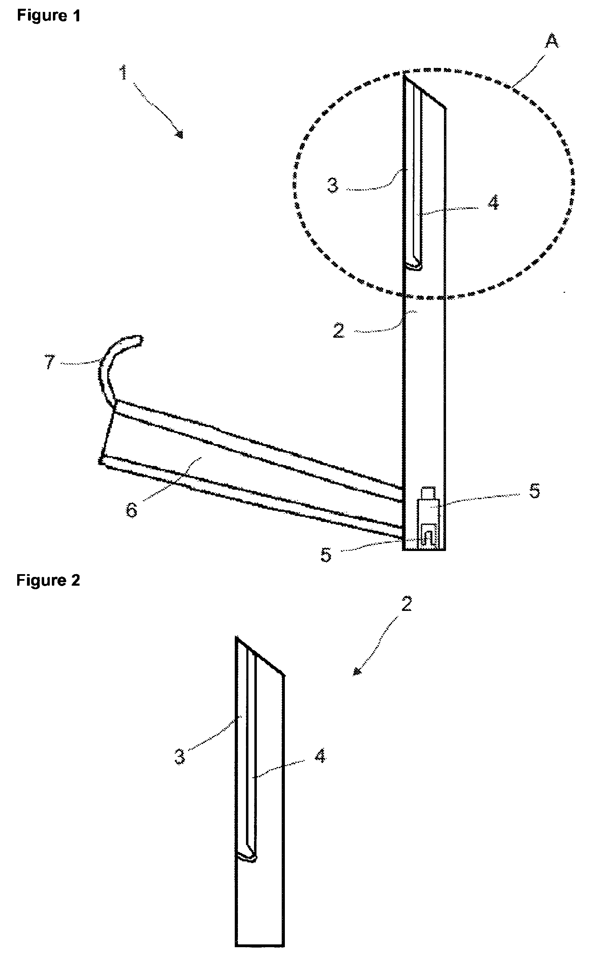

## Claims

1. A laryngoscope (1) which includes a body (2) which is cylinder in shape and empty inside with the tip adapted to be placed within the larynx
- at least one handle (6) fixed with one tip to the other end of the body (2) and
- at least one sleeve (7) which is fixed to the other end of the handle (6) unconnected to the body (2) and facilitates the motion of the body (2)
- the body (2) including; at least a canal (3) which runs along the outer wall of the body (2) starting from the tip adapted to be placed within the larynx towards its end having the handle (6) and upon which the intubation tube is placed; a ridge (4) running along the outer side of the canal (3) and adapted to prevent the slippage of the intubation tube within the larynx; and **characterised by** at least two burrows (5) located on the end of the body attached to the handle (6) and being adapted for placement of a light source and aspiration cannula within.

2. A laryngoscope (1) like in Claim 1 **characterized by the** canal (3) being as large as a portion of the surface of the intubation tube can lie upon, and which can be located on the left, right or midline of the lateral wall of the body (2) depending on usage.

3. A laryngoscope (1) like in Claim 1 or 2 **characterized by** the canal (3) being semicircular in shape.

4. A laryngoscope (1) like in any of the claims above **characterized by the** ridge (4) running along and being as long as the canal (3).

5. A laryngoscope (1) like in any of the claims above **characterized by the** ridge (4) the most superior end of which being on a level with the most superior end of the body (2), that is placed within the larynx.

6. A laryngoscope (1) like in any of the claims above **characterized by** the ridge (4) being adapted to fix the laryngeal part of the intubation tube placed within the canal (3) and to prevent the slippage of the intubation tube from over the body (2).

## Patentansprüche

1. Laryngoskop (1), das aufweist:
einen Körper (2), der zylinderförmig und innen leer ist, wobei ein Ende innerhalb des Kehlkopfs platziert wird,
mindestens einen Griff (6), der mit einer Spitze am anderen Ende des Körpers (2) befestigt ist, und
mindestens eine Hülle (7), die am anderen Ende des Griffs (6) befestigt ist, das nicht mit dem Körper (2) verbunden ist, und die Bewegung des Körpers (2) erleichtert,
wobei der Körper (2)aufweist: mindestens einen Kanal (3), der an der Außenwand des Körpers (2) entlang beginnend von der Spitze, die dazu angepasst ist, im Kehlkopf angeordnet zu werden, zu dessen Ende mit dem Griff (6) verläuft und auf dem der Intubationstubus platziert wird; einen Steg (4), der an der Außenseite des Kanals (3) entlang verläuft und dazu angepasst ist, das Abrutschen des Intubationstubus im Kehlkopf zu verhindern; und **gekennzeichnet durch** mindestens zwei Aushöhlungen (5), die am Ende des Körpers liegen, das am Griff (6) angebracht ist, und in denen eine Lichtquelle und eine Aspiratorkanüle platziert sind.

2. Laryngoskop (1) nach Anspruch 1, **gekennzeichnet durch** den Kanal (3), der so groß ist, dass ein Abschnitt der Oberfläche des Intubationstubus darauf liegen kann, und der links, rechts oder auf der Mittellinie der Seitenwand des Grundkörpers (2) gebrauchsabhängig angeordnet sein kann.

3. Laryngoskop (1) nach Anspruch 1 oder 2, **gekennzeichnet durch** den Kanal (3), der halbkreisförmig ist.

4. Laryngoskop (1) nach einem der vorstehenden Ansprüche, **gekennzeichnet durch** den Steg (4), der am Kanal (3) entlang verläuft und so lang wie dieser ist.

5. Laryngoskop (1) nach einem der vorstehenden Ansprüche, **gekennzeichnet durch** einen Steg (4), dessen oberstes Ende auf gleicher Höhe wie das oberste Ende des im Kehlkopf platzierten Körpers (2) liegt.

6. Laryngoskop (1) nach einem der vorstehenden Ansprüche, **gekennzeichnet durch** einen Steg (4), der dazu angepasst ist, den Kehlkopfteil des im Kanal (3) platzierten Intubationstubus zu fixieren und der das Abrutschen des Intubationstubus aus der Lage über dem Körper (2) verhindert.

## Revendications

1. Un laryngoscope(1) comportant :
Un corps (2) en forme cylindrique et vide à l'intérieure avec un bout adapté à être placé dans le larynx
- Au moins une poignée (5) fixée par un bout à l'autre extrémité du corps (2) et
- Au moins un manchon (7) fixé à l'autre extrémité de la poignée (6) non liée au corps (2) et facilitant le mouvement du corps (2)
- Le corps comportant au moins un canal (3) s'étendant le long de la paroi extérieure du corps (2) commençant du bout adapté à être dans le larynx vers son extrémité ayant une poignée (6) et sur laquelle est placée le tube d'intubation ; une crête s'étendant le long du côté extérieure du canal (3) et adaptée à prévenir le glissement du tube d'intubation dans le larynx ; et **caractérisé par** au moins deux logements (5) situés sur l'extrémité du corps attaché sur la poignée (6) et étant adapté pour le placement d'un source lumineuse et la canule d'aspiration à l'intérieur.

2. Un laryngoscope (1) selon la revendication 1, **caractérisé par** le canal (3) étant aussi large qu'une portion de la surface du tube d'intubation pourrait s'étendre au-dessus, et qui peut être placé sur la gauche, le droit, ou la ligne médiane de la paroi latérale du corps (2) dépendant de l'usage.

3. Un laryngoscope (1) selon les revendications 1 ou 2, **caractérisé par** le canal (3) en forme semi-circulaire.

4. Un laryngoscope (1) selon l'une quelconque des revendications ci-dessus, **caractérisé par** une crête s'étendant et étant aussi longue que le canal (3).

5. Un laryngoscope (1) selon l'une quelconque des revendications ci-dessus, **caractérisé par** une crête dont l'extrémité la plus supérieure étant au niveau avec l'extrémité le plus supérieure du corps (2) placé dans le larynx.

6. Un laryngoscope (1) selon l'une quelconque des revendications ci-dessus, **caractérisé par** une crête (4) adaptée à la partie laryngienne du tube d'intubation placé dans le canal (3) et à prévenir le glissement du tube d'intubation par-dessus du corps (2).
